Europäisches Patentamt

European Patent Office

Office européen des brevets

(11) Veröffentlichungsnummer: **0 274 657**
**A2**

## (12) EUROPÄISCHE PATENTANMELDUNG

(21) Anmeldenummer: **87118012.1**

(51) Int. Cl.4: **C07D 233/58**

(22) Anmeldetag: **05.12.87**

(30) Priorität: **12.12.86 DE 3642484**

(43) Veröffentlichungstag der Anmeldung:
**20.07.88 Patentblatt 88/29**

(84) Benannte Vertragsstaaten:
**AT BE DE ES FR IT**

(71) Anmelder: **BASF Aktiengesellschaft**
**Carl-Bosch-Strasse 38**
**D-6700 Ludwigshafen(DE)**

(72) Erfinder: **Mesch, Walter, Dr.**
**Richinesstrasse 11**
**D-6700 Ludwigshafen(DE)**

(54) Verfahren zur Herstellung von 4-methylimidazol.

(57) Verfahren zur Herstellung von 4-Methylimidazol durch Umsetzung von Hydroxipropanon und Formamid, wobei man die Umsetzung in Gegenwart einer mindestens doppelt molaren Menge Ammoniak, bezogen auf das Hydroxipropanon, in flüssiger Phase bei Temperaturen von 100 bis 200°C und Drücken von 10 bis 250 bar vornimmt.

EP 0 274 657 A2

## Verfahren zur Herstellung von 4-Methylimidazol

Diese Erfindung betrifft ein neues Verfahren zur Herstellung von 4-Methylimidazol aus Hydroxipropanon, Formamid und Ammoniak.

4-Methylimidazol, ein wichtiges Zwischenprodukt z.B. für die Herstellung von Endstoffen mit pharmakologischer Wirksamkeit, wird technisch durch Umsetzung von Methylglyoxal, Formaldehyd und überschüssigem Ammoniak hergestellt. Nach diesem Verfahren, das dem Syntheseweg nach Radziszewski (Chem.Ber. 2706 (1882)) entspricht, erhält man nur mäßige Ausbeuten.

Versucht man, 4-Methylimidazol entsprechend der aus Angew.Chem. 71 (1959), S. 759 bekannten Imidazolsynthese aus $\alpha$-Hydroxiketon und Formamid herzustellen, so läßt sich 4-Methylimidazol nur in untergeordneten Mengen gewinnen.

Es wurde nun überraschend gefunden, daß man 4-Methylimidazol in hoher Ausbeute und Reinheit erhält, wenn man die Umsetzung von Hydroxipropan und Formamid in Gegenwart einer mindestens doppelt molaren Menge Ammoniak, bezogen auf das Hydroxipropanon, in flüssiger Phase bei Temperaturen von 100 bis 200°C und Drücken von 10 bis 250, vorzugsweise 20 bis 50 bar vornimmt.

Nach dem Verfahren der Erfindung bemißt man die Mengenverhältnisse der Reaktanten so, daß man, bezogen auf 1 Mol Hydroxipropanon, 1 bis 10, vorzugsweise 1 bis 8, insbesondere 1 bis 4 Mol Formamid und mindestens 2 Mol, vorzugsweise mindestens 4 Mol, insbesondere mindestens 10 Mol, z.B. 50 bis 90 Mol, Ammoniak einsetzt. Da man die Umsetzung vorzugsweise in flüssigem Ammoniak vornimmt, kann die Ammoniakmenge z.B. bis zu 100 Mol, bezogen auf 1 Mol Hydroxipropanon, betragen.

Man führt die Umsetzung bei Temperaturen von 100 bis 200, vorzugsweise 140 bis 180°C durch. Der Dampfdruck des flüssigen Ammoniaks in der Mischung bedingt die Durchführung der Umsetzung in einem auf Druck ausgelegten Reaktor. Die sich dabei einstellenden Drücke betragen 10 bis 250, insbesondere 20 bis 50 bar. Die Reaktionszeiten liegen bei 30 bis 120 Minuten. Bei der diskontinuierlichen Arbeitsweise verwendet man einen Autoklav. Die bevorzugte kontinuierliche Umsetzung der drei Stoffströme wird z.B. in einem Druckrohr vorgenommen. Den Ammoniak gibt man in flüssiger Form unter Druck zu. Man kann aber auch die beiden anderen Komponenten für sich oder gemeinsam zum flüssigen Ammoniak geben.

Das Reaktionsgemisch wird z.B. durch fraktionierte Destillation aufgearbeitet.

Nach dem neuen Verfahren erhält man 4-Methylimidazol auf technisch einfache Weise in hoher Ausbeute und Reinheit.

Beispiel 1 (Vergleich)

In einen Rührkolben werden 270 g Formamid (6 g-Mol) gegeben. Man erhitzt auf 140°C und tropft unter Rühren 148 g Hydroxipropanon (2 g-Mol) hinzu. Das Reaktionsgemisch wird 3 Stunden gerührt. Man erhält ein dunkelbraunes Reaktionsprodukt, das gemäß gaschromatographischer Analyse 7 g 4-Methylimidazol (entsprechend 4 % d.Th.) enthält.

Beispiel 2 (Vergleich)

270 g Formamid (6 g-Mol) werden in einem Rührkolben auf 140°C erhitzt. Bei einem Druck von 150 mbar werden 148 g Hydroxipropanon (2 g-Mol) zugetropft. Gleichzeitig werden über eine Fraktionierkolonne 60 g Wasser im Gemisch mit etwas Hydroxipropanon abgezogen. Man erhält ein dunkelbraunes Reaktionsprodukt, das gemäß gaschromatographischer Analyse 8 g 4-Methylimidazol (entsprechend 5 % d.Th.) enthält.

Beispiel 3

270 g Formamid (6 g-Mol) und 148 g Hydroxipropanon (2 g-Mol) werden in einem 2 l-Rührautoklaven vorgelegt. Dann werden 340 g Ammoniak (20 g-Mol) flüssig zugepumpt. Beim Aufheizen auf 140°C stellt sich ein Druck von 50 bar ein. Diese Temperatur wird für 3 Stunden gehalten. Der Autoklav wird dann entspannt und das erhaltene Reaktionsgemisch über Kopf destilliert. Bei 10 mbar gehen zwischen 130 und 200°C 290 g einer hellgelben Fraktion über. Sie enthält nach gaschromatographischer Analyse 60 g 4-Methylimidazol, entsprechend 37 % d.Th.

Beispiel 4

In einem Röhrenreaktor von 1 l-Inhalt werden bei einem Durchfluß von unten nach oben gleichzeitig 200 ml/Std. eines Gemisches aus 32 g Hydroxipropanon (0,43 g-Mol) und 153 g Formamid (3,4 g-Mol) sowie 800 ml Ammoniak flüssig (47 g-Mol) gepumpt. Das Reaktionsgemisch wird auf 160°C gehalten. Der Reaktoraustrag wird durch Erwärmen auf 100°C ammoniakfrei gestellt. 1000 g des so erhaltenen Rohprodukts liefern bei der Destillation über Kopf ein hellgelbes Destillat mit einem Gehalt von 127 g 4-Methylimidazol. Dies entspricht einer Ausbeute von 81 % d.Th.

Wird der Rohaustrag über eine 30 cm-Füllkörperkolonne rektifiziert, so erhält man bei einer Übergangstemperatur von 136 bis 140°C und 12 mbar das 4-Methylimidazol in Form farbloser Kristalle vom Schmelzpunkt 51°C.

## Ansprüche

1. Verfahren zur Herstellung von 4-Methylimidazol durch Umsetzung von Hydroxipropanon und Formamid, dadurch gekennzeichnet, daß man die Umsetzung in Gegenwart einer mindestens doppelt molaren Menge Ammoniak, bezogen auf das Hydroxipropanon, in flüssiger Phase bei Temperaturen von 100 bis 200°C und Drücken von 10 bis 250 bar vornimmt.

2. Verfahren nach Anspruch 1, dadurch gekennzeichnet, daß man die Umsetzung bei Temperaturen von 140 bis 180°C und Drücken von 20 bis 50 bar vornimmt.

3. Verfahren nach Anspruch 1, dadurch gekennzeichnet, daß man je Mol Hydroxipropanon 1 bis 10 Mol Formamid anwendet.

4. Verfahren nach Anspruch 1, dadurch gekennzeichnet, daß man je Mol Hydroxipropanon 1 bis 8 Mol Formamid anwendet.

5. Verfahren nach Anspruch 1, dadurch gekennzeichnet, daß man je Mol Hydroxipropanon mindestens 4 Mol Ammoniak anwendet.

6. Verfahren nach Anspruch 1, dadurch gekennzeichnet, daß man je Mol Hydroxipropanon mindestens 8 Mol Ammoniak anwendet.

7. Verfahren nach Anspruch 1, dadurch gekennzeichnet, daß man je Mol Hydroxipropanon mindestens 10 Mol Ammoniak anwendet.